# EUROPEAN PATENT SPECIFICATION

(11) **EP 3 570 815 B1**
(45) Date of publication and mention of the grant of the patent: **03.04.2024**
(21) Application number: 18706571.9
(22) Date of filing: 22.01.2018
(51) Int. Cl.: A61K 9/00, A61K 47/18, A61K 9/08, A61K 47/36, A61K 47/38, A61K 38/40

(54) **COMPOSITION FOR OPHTHALMIC USE**
ZUSAMMENSETZUNG ZUR OPHTHALMISCHEN VERWENDUNG
COMPOSITION À USAGE OPHTALMIQUE

(30) Priority: 20.01.2017 IT 201700006360
(43) Date of publication of application: 27.11.2019
(73) Proprietor: Italdevice S.r.l., 00040 Pomezia (RM) (IT)
(72) Inventor: TIBERI, Licia, 00040 Pomezia (RM) (IT)
(74) Representative: Benedetto, Marco
(86) International application number: PCT/IB2018/050380
(87) International publication number: WO 2018/134792

(56) References cited:
- EP-A2- 1 055 720
- WO-A1-2014/192068
- WO-A2-03/079997
- JP-A- 2007 277 153
- Anonymous: "MFDS - Ministry Of Food And Drug Safety - BIO & COSMETICS > Approval Process > Quasi-Drugs", Internet , 11 April 2018 (2018-04-11), XP55466202, Internet Retrieved from the Internet: URL:http://www.mfds.go.kr/eng/index.do?nMe nuCode=169 [retrieved on 2018-04-11]
- "Aq. Eye lotion having good stability", WPI WORLD PATENT INFORMATION DERWENT, vol. 27, no. 95, 9 May 1995 (1995-05-09), XP002063116,

## Description

The present invention relates to a composition for ophthalmic use comprising hydroxypropyl methylcellulose and lactoferrin and the use thereof for the curative or preventive treatment of disorders or pathologies of the eye and of the periocular area.

The ocular surface is a delicate structure, whose functioning depends on a series of systems that contribute to its physiological integrity. The integrated functions of the various components of the ocular surface must work in an optimal manner.

The ocular surface is composed of the superficial and glandular epithelium of the cornea, conjunctiva, tear gland, accessory tear glands, meibomian gland, associated glands of Moll and Zeiss, and nasolacrimal duct. These components are conceived as a single functional system.

The tear film consists of an aqueous layer secreted by the tear glands, a mucosal layer produced by the goblet cells of the conjunctiva and epithelial cells of the conjunctiva and cornea, and a lipid layer secreted primarily by the meibomian glands.

Each structure making up the system of the ocular surface contributes to the composition and efficiency of the tear film, which serves to keep the eye hydrated and to protect it from potential damage caused by external factors. In fact, the ocular surface is the mucosal surface of the body most exposed to the hazards of the environment. Among the various environmental factors that expose the eye to potential surface damage, it is possible to mention wind, ultraviolet radiation and polluting agents.

These environmental factors, by acting on the ocular surface, can give rise to symptoms of various kinds (sensation of tired eyes, redness, associated with itching, burning, foreign body sensation and irritation), give rise to dry eye syndrome due to environmental causes of a transitory type (as in the case of ultraviolet radiation), trigger a dry eye syndrome due to environmental causes, aggravate the symptoms of a dry eye syndrome due to pre-existing environmental causes and be a cause of conjunctivitis (e.g. of the irritative type).

In general, a dry environment can produce an increase in the evaporation of the tear film and/or a worsening of its turnover and clearance.

A dry climate, wind, ultraviolet radiation (UV) and pollution are factors that determine tear film instability, with a consequent inflammation of the ocular surface.

Dry eye syndrome can be episodic and become symptomatic, for example, when environmental factors cause tear film instability. In the initial stages, the symptoms can be mild, visual fatigue (tired eyes) being included among the symptoms. Since the tear film in patients with eye dry is unstable and incapable of maintaining the protective qualities that are necessary for the eye's structure and function, patients complain of symptoms of discomfort such as burning, pin prick sensation, grittiness, foreign body sensation, lacrimation, eye fatigue and dryness.

The ocular surface is naturally colonised by bacteria that are acquired from birth and which, in the course of a lifetime, can undergo changes in quality and quantity. These microorganisms represent the normal microbial population, i.e. the resident microbiota. Under normal conditions this system is in equilibrium and balanced. There are mainly three factors that regulate this equilibrium and modify it: tissue-related, microbial and environmental factors.

The first are dependent on the histological structure of conjunctival tissue, the secretions making up the tear film, and the presence of antigenically competent cells with phagocytic activity.

The microbial factors are specific factors of pathogenicity, characteristic of several microbial species. The third ecosystem-regulating factor, which renders the pathogenesis of conjunctivitis more of a particular concern today and has made these conditions increasingly difficult to resolve therapeutically, is represented by individual and environmental factors: severe visual deficits, incorrect therapeutic use of ocular prostheses, air, water and lead pollution, pollution from exhaust gases and industrial products and ergophthalmological pathology.

Furthermore, the eye, like all other structures of the body that are in contact with the outside environment, is often vulnerable to the attack of microorganisms with pathogenic action, responsible for infections of varying severity and extension.

On reaching the ocular surface, microorganisms tend to persist and adhere to the conjunctiva and, in general, to stabilise and colonise on a tissue through a dynamic process marked by a high or modest adhesion capacity, irrespective of the characteristics of virulence. The phenomenon of adherence, which results in the multiplication of bacteria, must be considered as the initial moment of colonisation. It gives rise to the formation of micro colonies, which indicate that an environmental optimum has been reached by the bacterial species that succeeds in multiplying itself, colonising the surface of the cells and remaining stably on that type of tissue. The appearance of micro colonies indicates that the microorganisms have succeeded in organising themselves, creating a particular habitat of their own and stabilising themselves permanently, forming a complex biofilm. In this situation the microorganisms remain stably on the conjunctival surface. The complexity and dynamism of the ecosystem is further maintained by the fact that the micro colonies exert a "trapping" effect on nutrients, absorbing certain substances, such as ionised iron, which makes the metabolic situation of the microorganisms on that surface even more optimal. Metabolic products of digestion or of the chemical modification of simple molecules subsequently begin to appear, e.g. polysaccharides and other macromolecules, which render the ecosystem even more complex. Highly defined situations develop and, in this manner, a protective barrier is established around the bacterial colonies, which become impervious to many antibacterial substances and even antibiotics which, despite demonstrating excellent activity in vitro on the same bacterial strains, prove to be ineffective in vivo.

Another interesting aspect of a bacterial ecosystem is that this type of complexing of microbiota with secretory components does not stabilise only on skin surfaces and mucosae, but also on biomaterials, in particular on the polymers used to manufacture prostheses and contact lenses. In fact, bacteria are in a condition to adhere to and stabilise on biopolymers and to construct completely organised micro colonies and biofilms. In this case as well, the action of macrophages is reduced or impeded, because they are unable to arrive at this level and penetrate the barrier, so they act only on the so-called planktonic forms, i.e. microorganisms that are still partially free and not completely adherent and enclosed in the biofilm. Similarly, antibiotics and antibodies as well are able to modify the biofilm and the complex macromolecular organisation of the ecosystem to a very small extent, once the latter has stabilised.

An increasing amount of scientific evidence demonstrates that bacterial biofilm plays a key role in eye infections. Bacterial growth is characterised as a biofilm when the bacteria attach to one another or to a surface.

Biofilm formation is a process that is genetically part of the bacteria life cycle, as a consequence of numerous modifications in the cellular physiology of the organism, and this has resulted in growing resistance to antibiotics. The presence of bacterial biofilm has been demonstrated on many medical devices, such as contact lenses, scleral buckles, suture materials and intraocular lenses. Many eye infections occur precisely when these devices come into contact with the eyes or are implanted therein.

Another type of eye infection is endophthalmitis, a posterior eye infection caused by the physical introduction of bacteria into the eye during a surgical intervention or as a result of trauma, or through contamination from a site of infection located in other regions and which can occasionally reach the eye through the systemic circulation.

Several scientific studies have demonstrated that biofilm plays a key role also in this type of infection, especially in postoperative or trauma-induced endophthalmitis.

Similar situations are confronted in veterinary practice.

The majority of ophthalmic pathologies that present themselves in veterinary practice regard the outer portion of the eye, with a prevalence of corneal diseases.

The cornea of domestic animals is composed of 4 layers: epithelium, stroma, Descemet's membrane and endothelium. Bowman's membrane, present in primates, and Dua's layer, discovered only recently, do not exist in domestic animals or have not yet been described. The outermost layer of the cornea consists of the epithelium, in turn composed of 5-7 layers divided into 3 different areas: on the exterior the flattened superficial cells with microvilli for ensuring good adhesion of tear fluid, in the middle the intermediate wing cells, and in the lowest layer the basal cells, closely connected with the basement membrane through hemidesmosomes. The stroma, by contrast, accounts for about 90% of the thickness of the cornea. This layer is composed mainly of keratocytes, nerve fibres and collagen fibrils inserted in a matrix with a high content of glycosaminoglycans. The regular, parallel arrangement of these collagen fibrils, with a thickness of only 25 nm at a distance of about 1 nm, the absence of blood vessels and pigmentation, the unhardened state of the superficial epithelial cells and the loss of the myelin sheath of corneal nerves are the necessary conditions for assuring the transparency of the cornea. Finally, Descemet's membrane constitutes the extremely coriaceous transparent basement membrane of the innermost layer of the cornea, the endothelium. The endothelium is a single cellular layer composed of hexagonal cells endowed with coupled Na/K ATPase pumps that serve to decongest the corneal stroma, maintaining the parallel arrangement of collagen fibres. Corneal alterations are often classified on the basis of their ulcerative or non-ulcerative effects. In the case of ulcerative modifications, there is a loss of corneal substance, which is in turn classified according to depth and cause. Whereas an intact corneal epithelium is very resistant against bacterial infections, following a loss of corneal substance, bacteria can easily establish themselves and proliferate. Toxins and metalloproteinase, of both bacterial and leukocytic origin, can lead, in the space of just a few hours, to the dissolution of collagen fibrils in the corneal stroma with a consequent perforation of the cornea and potentially even loss of the eye. In addition to targeted antibiotic therapy (possibly based on an antibiogram), great importance should be attributed to the inhibition of these proteases. To this end, from a clinical standpoint, use is made above all of tetracyclines (also systemic), N-acetylcysteine, ethylenediaminetetraacetic acid disodium salt (Na-EDTA) and a2- macroglobulin.

WO 03/07997 A2 discloses an ophthalmic formulation for the treatment of glaucoma and intraocular pressure with a prostaglandin compound of the F-series (PGF) wherein the preservative is an antimicrobial peptide, such as lactoferrin.

At present, there are no available compositions for ophthalmic use that are capable of assuring, simultaneously, optimal lubrication of the eye and an effective antibacterial action, also because of the growing resistance of biofilms to traditional antibiotics, as described above.

It is necessary to highlight that incompatibilities have been found between the wide-spectrum antibiotics generally used in the ophthalmic field and some of the substances used to improve lubrication of the eye or maintain it in an optimal condition. Such incompatibilities, due to the chemical structure of the substances, their physical properties or unknown causes, also lead to an instability of the preparations which limits their shelf life.

One object of the present invention is to provide preparations for ophthalmic use which are able to ensure optimal lubrication of the eye and effective antibacterial action.

Another object of the present invention is to provide a composition for ophthalmic use that can be used in the prevention and/or treatment of ocular pathologies such as conjunctivitis or bacterial infections and conditions such as dry eye, occurring as a consequence of bacterial infections and/or conjunctivitis, also for users of devices such as contact lenses and scleral buckles.

Another object of the present invention is to provide a composition for ophthalmic use which, in addition to the above-mentioned advantages, is stable and has an acceptable shelf life.

These objects, and still others, which will become apparent from the detailed description that follows, are achieved by the composition of the present invention thanks to the features specified in the appended claims. The composition for use of the present invention has valid application for administration to a subject on the ocular surface or in the periocular area.

The invention is defined by the claims, which disclose compositions containing a mixture of HPMC, lactoferrin and EDTA. Any subject-matter falling outside the scope of the claims is provided for information purposes only.

The composition according to the present invention can be advantageously used in the curative and/or preventive treatment of disorders or pathologies such as conjunctivitis and blepharitis, as a mild antibiotic in the absence of other antibiotics, or as an adjuvant to antibiotic therapy. The substances generally used as antibiotics have difficulty in destroying bacterial biofilm, whereas it has been found that the composition of the present invention, in particular in the presence of EDTA, effectively reduces, or eliminates, the biofilm responsible for infections that cause conjunctivitis and blepharitis.

The subject matter of the present description relates to a composition (C) for ophthalmic use comprising a mixture which comprises or, alternatively, consists of:
(a) hydroxypropyl methylcellulose and/or xanthan gum,
(b) lactoferrin or derivatives thereof, and
(c) one or more excipients for pharmaceutical use, with the exclusion of ethylenediaminetetraacetic acid (EDTA).

The subject matter of the present description further relates to a pharmaceutical form or a medical device comprising the composition (C) as defined above.

The subject matter of the present description further relates to (1) the non-therapeutic use of the composition (C) as defined above to attenuate conditions such as dry eye or fatigue, wherein said use comprises administration of the composition (C) to a subject on the ocular surface or in the periocular area, and (2) the composition (C) as defined above for use in the preventive and/or curative treatment of pathologies of the eye or of the periocular area, wherein said use comprises administration of the composition (C) to a subject on the ocular surface or in the periocular area.

The subject matter of the present invention relates to a composition (C1) for use in the curative or preventive treatment of at least one pathology or disorder of the eye or of the periocular area, and of the symptoms thereof comprising:
i. a mixture which comprises or, alternatively, consists of:
   (a) hydroxypropyl methylcellulose ,
   (b) lactoferrin, and
   (c) ethylenediaminetetraacetic acid (EDTA) or a salt thereof, and, optionally,
ii. one or more excipients for pharmaceutical use;

wherein said at least one pathology or disorder is at least one among conjunctivitis of bacterial origin, blepharitis, a bacterial infection and dry eye syndrome caused by bacteria belonging to the *species Staphylococcus* aureus or *Pseudomona aeruqinosa,* and
wherein said use comprises administration of said composition to a subject on the ocular surface or in the periocular area.

The subject matter of the present description further relates to a pharmaceutical form or a medical device comprising the composition (C1) as defined above.

The subject matter of the present description further relates to the non-therapeutic use of the composition (C1) as defined above to attenuate conditions such as dry eye or fatigue, wherein said use comprises administration of said composition to a subject on the ocular surface or in the periocular area, with the exclusion of the use of a composition in the form of a cream comprising lactoferrin and EDTA and xanthan gum as component (a).

The subject matter of the present invention further relates to the use of a composition comprising:
i. a mixture comprising, or consisting of
   (a) hydroxypropyl methylcellulose;
   (b) lactoferrin f;
   (c) ethylenediaminetetraacetic acid (EDTA) or a salt thereof, and, optionally,
ii. one or more excipients for pharmaceutical use,
to prevent or reduce the formation of biofilm caused by bacteria belonging to the species *Staphylococcus aureus* or *Pseudomona aeruginosa* on contact lenses.

The present description relates to a method for the curative or preventive treatment of at least one pathology or disorder of the eye or of the periocular area, and of the symptoms thereof, wherein said treatment comprises administration to a subject, on the ocular surface or in the periocular area, of a composition (C1) comprising:
i. a mixture which comprises, or, alternatively, consists of:
   (a) hydroxypropyl methylcellulose and/or xanthan gum;
   (b) lactoferrin or derivatives thereof;
   (c) ethylenediaminetetraacetic acid (EDTA) or a salt thereof; and, optionally,
ii. one or more excipients for pharmaceutical use.

Advantageously, the composition (C) of the present description and composition (C1) according to the present invention can be used in humans or for veterinary use, by optionally modifying the formulation in an appropriate manner, as known to the person skilled in the pharmaceutical art in the human and veterinary fields.

In the context of the present invention, "periocular area" means the part of the human or animal body that is located around the ocular surface as defined above, i.e. which forms the eye contour. By way of non-limiting example, the periocular area, as understood here, is made up of the eyelids, eyelashes, the skin of the periorbital area and all tissues connected to or near the ocular surface.

The present composition is to be understood as for human or veterinary use indistinctly, i.e. as a preparation to be applied to animals with the uses and methods known to the person skilled in the art. In the context of the present invention, "treatment" means an intervention comprising the administration of a substance, or mixture of substances, having the aim of eliminating, reducing or preventing a pathology and the symptoms thereof.

Unless specified otherwise, the content of an ingredient in a composition relates to the percentage by weight of that ingredient relative to the total weight of the composition.

Unless specified otherwise, the indication that a composition "comprises" one or more components means that other components may be present in addition to the one or ones specifically indicated, even though they need not necessarily be indicated, i.e. the composition may also contain exclusively those components, and the indication that a composition "consists" of given components means that the presence of other components is ruled out.

The inventor has found that a composition for ophthalmic use comprising a mixture (combination) of lactoferrin and hydroxypropyl methylcellulose (hypromellose) has an excellent stability and shelf life. In contrast, the combination of lactoferrin with other polymers known as lubricants for ophthalmic use, such as, for example, hyaluronic acid, carboxymethyl cellulose and hydroxylethyl cellulose, has given unsatisfactory results in terms of stability, since a substantial degradation of the lactoferrin and/or of the polymer used has been observed, along with the formation of undesirable byproducts.

The mixture contained in said composition (C) or (C1) of the present invention, comprising a combination of lactoferrin, EDTA and hydroxypropyl methylcellulose (hypromellose) according to the invention thus makes it possible to ensure excellent lubrication of the ocular surface and to prevent or treat bacterial infections, together with excellent stability of the active ingredients and an excellent shelf life.

In one embodiment of the description, the component (a) of the composition (C) or (C1) according to the invention is xanthan gum.

Xanthan gum (also indicated as xanthan) is a polysaccharide used as a food additive and rheological modifier. On the labels of food products, it is indicated as E415 and its CAS Number is 11138-66-2. Xanthan gum is obtained through a process of fermentation, in a pure culture of a carbohydrate (glucose or sucrose), by natural strains of the bacterium *Xanthomonas campestris,* purified by extraction with ethanol or 2-propanol, dried and ground. It contains D-glucuronic and pyruvic acids and, as the main hexoses, D-glucose and D-mannose, and is prepared in the form of sodium, potassium or calcium salts. The pH of a 1% aqueous solution is comprised between 5.5 and 8. Xanthan gum appears as a cream-coloured powder that is soluble in water and insoluble in ethanol. The xanthan gum in the composition according to the invention can also be prepared in the form of a sodium, potassium or calcium salt.

The xanthan gum for use in the composition (C) or (C1) according to the present invention preferably has a particle size of 100 to 500 mesh, more preferably 200 mesh to 400 mesh, and/or a viscosity (1 % solution in 1 % KCI in water) of 1000 to 2000 cPs, preferably 1200 to 1600 cPs.

The component (a) of the composition (C) or (C1) according to the invention is hydroxypropyl methylcellulose.

Hydroxypropyl methylcellulose, or methylhydroxypropylcellulose, often abbreviated as hypromellose or HPMC, is a semi-synthetic inert viscoelastic polymer used as an ophthalmic lubricant, as well as an excipient and component for controlled delivery in oral medications, and is used in a variety of commercial products. Methylhydroxypropylcellulose is a cellulose in which some hydroxyl groups are substituted by methyl ethers and some by 2-hydroxypropyl ethers. Its Codex Alimentarius (E number) is E464 and its CAS number is 9004-65-3. The presence of the hydroxyl groups in a different percentage determines the apparent viscosity, calculated in mPa s (millipascals per second) in a 2% solution at 20°C. Methylhydroxypropylcelluloses are identified by a number related to their viscosity. In the USA they are distinguished by a 4-digit number, the first two of which represent, in percentage terms, the approximate content of the methoxyl groups and the last two the approximate content of the hydroxypropyl groups. It appears in the form of granules or a white, yellowish-white or greyish-white fibrous powder, which is practically odourless, hygroscopic after drying and soluble in cold water with the formation of a colloidal solution.

In a preferred embodiment, the hydroxypropyl methylcellulose according to the present invention has a percentage of methoxyl groups comprised from 25% to 35%, preferably 28% to 30%, and/or a percentage of hydroxypropoxyl groups comprised from 5% to 15%, preferably 7% to 12% or 9% to 10%, wherein the percentage is calculated on the basis of the total hydroxyl groups present, free and etherified, according to methods known to the person skilled in the art, for example by means of those of the monograph for hypromellose in the US Pharmacopeia, 2013 edition (official 1 May 2014), hereinafter USP.

In a preferred embodiment, the hypromellose according to the present invention has a viscosity (2% solution) comprised from 2700 mPas to 5000 mPas, preferably 2800 mPas to 4000 mPas or 3000 mPas to 3500 mPas, according to the method provided in the USP as identified above.

Lactoferrin (component (b) contained in the composition (C) or (C1) according to the present invention), also known as lactotransferrin, LTF, GIG12, HEL110, HLF2 and LF, is a multifunctional globular protein with antimicrobial activity, both bactericidal and fungicidal.

Lactoferrin belongs to the family of transferrins and possesses a molecular mass of 80 KDa, with two binding sites for the ferric ion (Fe³⁺ ), like transferrin itself. Lactoferrin is never iron saturated and its ferric content varies. Lactoferrin is an excellent adjuvant to antibiotic therapy, as it is endowed with marked antibacterial and antifungal properties and, therefore, where an antibiotic does not work because of the numerous resistances that prolonged and improper use has generated, using a product that aids in that activity, and is also free of side effects, is surely advantageous. Nearly all the families of antibiotics present today on the pharmaceutical market are susceptible to the occurrence of resistant strains. Moreover, such strains, create a protective biofilm, which becomes practically invulnerable to the antibiotic. Lactoferrin, on the other hand, by acting with a sequestration mechanism, deprives bacteria of the elements they need to reproduce. In addition to this, lactoferrin is also endowed with its own antibacterial activity against some iron-independent species; in fact, some studies have demonstrated its interaction with the lipopolysaccharides of bacteria, above all negative ones. It also has an antifungal activity and interrupts the replication mechanism of some viruses.

Lactoferrin can be used for the treatment of disorders and ocular pathologies, although its unsatisfactory stability in ophthalmic formulations, because of incompatibility with some substances such as ocular lubricants, has limited its use.

The use of lactoferrin in combination with EDTA was described in Payne, C. et al. Journal of Food Protection, Number 1 , January 1994, pp. 4-86, pp. 62-65(4), Nielsen, N. et al. J. Agric. Food Chem., 2004, 52 (25), pp 7690-7699, and del Olmo, A. et al. J Food Prot. 2009 Apr;72(4):760-5 for the inhibition of potentially pathogenic bacterial strains present as contaminants in foods, such as *Pseudomonas fluorescens, Salmonella typhimurium, Escherichia coli* O157: H7, and *Listeria monocytogenes,* with results varying based on the specific bacterial strains. Payne et al. indicate that lactoferrin and EDTA, on their own or in combination, influence neither the growth of *Pseudomonas fluorescens* nor that of *Salmonella typhimurium* and del Olmo et al. report that the activity of lactoferrin and EDTA on the growth of *Pseudomonas fluorescens* in ground meat is influenced by many factors tied to the substrate.

The lactoferrin for use in the composition (C) or (C1) according to the present invention can be of various origins, including, for example, human, bovine, swine and equine, and from genetically modified organisms, as well as different biological secretions such as, for example, colostrum, or milk from every stage of lactation, or processed or semi-processed products such as, for example, buttermilk or skimmed milk.

The subject matter of the description also relates to compositions comprising lactoferrin derivatives and analogues, such as, by way of non-limiting example, immobilised lactoferrin (e.g. US 6, 172,040), analogues thereof of synthetic origin, with a molecular weight lower than that of natural lactoferrin or conjugates thereof with other substances.

The composition (C) or (C1) according to the present invention optionally comprises at least one excipient for pharmaceutical use (component ii.) comprised among those known to the person skilled in the art and usable in liquid, semisolid or solid ophthalmic formulations, with the exclusion of EDTA, which is present in the composition (C1) and, optionally, in the composition (C) according to the present invention as an active ingredient.

By way of non-limiting example, the excipients that can be included as component ii. in the composition according to the present invention comprise preservatives, antioxidants, stabilisers, thickeners, rheology modifiers, biocides, colourants, pH buffers, soothing agents and anti-inflammatories.

As the excipient ii., the composition according to the present invention preferably comprises sodium hydroxymethylglycinate in aqueous solution at 50% by weight (MicroGlicin 50).

The composition (C) or (C1) according to the present invention further contains: (c) ethylenediaminetetraacetic acid (EDTA), or a mono-, di-, tri- or tetravalent salt thereof, such as disodium salt (CAS number 6381-92-6).

For the sake of clarity, in the context of the present invention, EDTA represents an active component of the composition and is not included in the definition of excipient.

It has been found that the combination of lactoferrin and EDTA produces a synergistic effect that reduces, or eliminates, the bacterial biofilm, comprising, for example, bacteria belonging to the species *Staphylococcus aureus* or *Pseudomona aeruginosa,* which is responsible for infections that can cause conditions such as conjunctivitis, blepharitis and dry eye, particularly when lactoferrin and EDTA are in a weight ratio close to 1 : 1 , e.g. comprised from 0.8: 1 to 1 :0.8.

In a preferred embodiment, the composition (C) or (C1) according to the invention comprises each of the components (a), (b), (c) and, if present, one or more excipients ii., each one independently of the other, in an amount of 0.05% to 1%, wherein all the percentages are by weight of the component relative to the total weight of the composition according to the invention.

In a more preferred embodiment, in the composition (C) or (C1) according to the invention, (a) is in an amount of 0.10% to 0.15%, (b) is in an amount of 0.10% to 0.15%, (c) is in an amount of 0.10 to 0.35%, and/or ii., if present, is in an amount of 0.10 to 0.35%, wherein all the percentages are by weight of the component relative to the total weight of the composition.

In a preferred embodiment, in the composition (C) or (C1) according to the invention, (a) is hydroxypropyl methylcellulose, (b) is lactoferrin and EDTA or a salt thereof is comprised.

In a preferred embodiment, the composition (C) or (C1) according to the invention further comprises glycyrrhizic acid, or a salt thereof, such as dipotassium salt.

Advantageously, it has been found that glycyrrhizic acid, or a salt thereof, has a synergistic effect with the other ingredients of the composition, which results in a faster and more effective preventive or curative treatment of ocular conditions such as dry eye or fatigue.

In a preferred embodiment, the present invention may relate to a composition (C1) for use in the curative or preventive treatment of at least one pathology or disorder of the eye or of the periocular area, and of the symptoms thereof for ophthalmic use, comprising:
i. a mixture which comprises or, alternatively, consists of:
   (a) hydroxypropyl methylcellulose and/or xanthan gum,
   (b) lactoferrin or derivatives thereof,
   (c) ethylenediaminetetraacetic acid (EDTA) or a salt thereof, and
   (d) glycyrrhizic acid, or a salt thereof,
ii. optionally, one or more excipients for pharmaceutical use,
wherein said use comprises administration of said composition to a subject on the ocular surface or in the periocular area.

In one aspect, the present invention provides a pharmaceutical form suitable for ophthalmic use or a medical device comprising the composition as defined above.

In the context of the present invention, the term "medical device" is used with the meaning according to Italian Legislative Decree no. 46 of 24 February 1997, which corresponds to the definition provided by the World Health Organization and available at the URL https://www.who.int/medical_devices/full_deffinition/en/, i.e. it indicates a substance or another product, used alone or in combination, intended by the manufacturer to be used in humans for the purposes of diagnosis, prevention, monitoring, treatment or alleviation of disease, which device does not achieve its primary intended action by pharmacological, immunological or metabolic means, in or on the human body, but which may be assisted in its intended function by such means.

In one embodiment, the pharmaceutical composition or medical device according to the present invention is in the form of a liquid preparation such as, for example, an aqueous or oily collyrium, eye drops or suspension, a semi-solid preparation such as, for example, an unguent, ointment, gel or cream, with the exclusion of a cream comprising lactoferrin, EDTA and xanthan gum (as component (a)), or a solid preparation such as, for example, a powder, capsule, compress, ocular insert and the like.

Disclosed, but not claimed, is the non-therapeutic use of the composition (C) or (C1) as defined above to attenuate conditions such as dry eye or fatigue, wherein said use comprises administration of said composition to a subject on the ocular surface or in the periocular area, with the exclusion of a composition in the form of a cream comprising lactoferrin, EDTA and xanthan gum (as component (a)).

In one aspect, the present invention provides the composition (C) or (C1) as described above for use in the treatment of pathologies of the eye or of the periocular area, wherein said use comprises administration of said composition to a subject on the ocular surface or in the periocular area, as defined above.

The subject matter of the present invention relates to a composition (C) or (C1) as defined above for use in the curative or preventive treatment of at least one pathology or disorder of the eye or of the periocular area such as ocular infections, wherein said use comprises administration of said composition to a subject on the ocular surface or in the periocular area, wherein said at least one pathology or disorder is at least one among conjunctivitis of bacterial or viral origin, blepharitis, a bacterial or viral infection and dry eye syndrome.

Said composition (C) or (C1) for use as defined above is preferably for use in the preventive treatment of said symptoms, which comprises administration of said composition without further substances with an antibiotic action, or said use for curative treatment comprises administration of (C) in combination with one or more substances with antibiotic action.

The composition (C) or (C1) according to the invention can be validly used in the treatment of so-called dry eye syndrome.

"Dry eye syndrome" is one of the most frequent pathologies in ophthalmology; its incidence is high in the middle-aged and elderly population and increases with age.

Correlated risk factors have shown to be: diabetes, allergy, and the intake of antihistamines, diuretics and corticosteroids.

The composition (C) or (C1) according to the present invention can be advantageously used also in conjunctivitis, above all of a bacterial or viral nature. Conjunctivitis is an inflammation that affects the conjunctiva, i.e. the membrane lining the eyelid, and the cornea, the white part of the eye, resulting in the classic characteristic appearance of red eyes. Conjunctivitis affects not only adults, but also children and infants.

A virus, bacterium, allergy or irritation caused by smoke or other polluting agents can cause conjunctivitis, which can also accompany a cold, an exanthematous disease (above all measles) or irritation due to exposure to very bright natural or artificial light.

Conjunctivitis can affect most adults and children and is in any case contagious, so that it would be advisable to undertake an effective therapy right away to avoid recourse to drugs.

Also, in the case of blepharitis, an inflammation of the eyelids, against which the composition according to the invention (C) or (C1) can be used, there are different intervening factors, such as:
- Bacterial infections sustained in particular by bacteria of the genus *Streptococcus*
- Viral infections sustained in particular by the type I *Herpes simplex* virus (ocular herpes);
- Allergies, including allergic reactions to eye drops, ophthalmic ointments, solutions for contact lenses and eye cosmetics;
- Acne Rosacea: a particular forma of chronic dermatitis characterised by the appearance of erythema, teleangectasia and furuncles on the face;
- Allergic, irritative or infectious conjunctivitis (inflammation of the conjunctiva that degenerates into blepharitis).

In the case of blepharitis induced by bacteria and viruses, the composition (C) or (C1) of the present invention can also be usefully employed, especially in sensitive individuals, as a preventive therapy. Finally, the composition (C) or (C1) of the present invention reduces the risk of biofilm formation on contact lenses and therefore reduces the risk of infections tied to the prolonged use of the same.

Embodiments FRn of the present description are set forth below.

In one embodiment (FR1), the present description relates to a composition (C) for ophthalmic use comprising a mixture which comprises, or, alternatively, consists of:
(a) hydroxypropyl methylcellulose and/or xanthan gum;
(b) lactoferrin or derivatives thereof;
(c) one or more excipients for pharmaceutical use, with the exclusion of ethylenediaminetetraacetic acid (EDTA).

In a preferred embodiment (FR2), the present description relates to the composition (C) according to (FR1), further containing (d) ethylenediaminetetraacetic acid (EDTA) or a salt thereof.

In a preferred embodiment (FR3), the present description relates to the composition (C) according to either (FR1) or (FR2), wherein (a) is hydroxypropyl methylcellulose.

In a preferred embodiment (FR4), the present description relates to the composition (C) according to any one of (FR1) - (FR3), which comprises or, alternatively, consists
of:
- (a) in an amount of 0.05% to 1%, preferably 0.10 to 0.15;
- (b) in an amount of 0.05% to 1%, preferably 0.10 to 0.15;
- (c) in an amount of 0.05% to 1 %, preferably 0.10 to 0.15; and/or
- (d) in an amount of 0.05% to 1 %, preferably 0.10 to 0.15, wherein all the percentages are by weight of the component relative to the total weight of the composition

In a preferred embodiment (FR5), the present description relates to the composition (C) according to any one of (FR1) - (FR4), which further comprises glycyrrhizic acid or salts thereof.

In a preferred embodiment (FR6), the present description relates to the composition (C) according to any one of (FR1) - (FR5), wherein (a) is hydroxypropyl methylcellulose, (b) is lactoferrin, (d) is EDTA or a salt thereof, and which optionally comprises glycyrrhizic acid or salts thereof.

In one embodiment (FR7), the present description relates to a pharmaceutical form suitable for ophthalmic use or a medical device suitable for ophthalmic use comprising the composition (C) according to one of the embodiments (FR1)-(FR6), preferably in the form of a liquid preparation such as, for example, an aqueous or oily collyrium, eye drops or suspension, a semi-solid preparation such as, for example, an unguent, ointment, gel or cream, or a solid preparation such as, for example, a powder, capsule, compress, ocular insert and the like.

In one embodiment (FR8), the present description relates to the composition (C) according to one of the embodiments (FR1)-(FR6) for use in the curative or preventive treatment of the symptoms of pathologies of the eye or of the periocular area, such as ocular infections, wherein said use comprises administration of the composition (C) to a subject on the ocular surface or in the periocular area.

In a preferred embodiment (FR9), the present description relates to the composition (C) for use according to the embodiment (FR8), wherein said use for preventive treatment comprises administration of (C) without further substances with antibiotic action or said use for curative treatment comprises administration of (C) in combination with one or more substances with antibiotic action.

In one embodiment (FR10), the present description relates to the non-therapeutic use of the composition (C) according to one of the embodiments (FR1)-(FR6) to attenuate conditions such as dry eye or fatigue, wherein said use comprises administration of said composition to a subject on the ocular surface or in the periocular area.

The following examples provide practical embodiments of the invention, without the intention of limiting the scope thereof.

### EXPERIMENTAL PART

Ingredients used:
- MicroGlicin 50 (Agrar S.r.L. Rome, Italy), 50% sodium hydroxymethylglycinate by weight in aqueous solution, CAS No. 70161-44-3;
- EDTA (Sigma-Aldrich): ethylenediaminetetraacetic acid, sodium salt dihydrate, purity 99.0-101.0%, CAS number 6381-92-6;
- Hydroxypropyl methylcellulose (Agrar S.r.L. Rome, Italy), medium viscosity, CAS number 9004-64-3, pH 7.3, corresponds to USP, NF and FCCC latest editions, methoxyl 28.0-30.0, hydroxypropyl 7.0-12.0%, 2% viscosity solution 2700-5000 mPas, particle size: 99.9% less than 400 micrometres.
- 95% lyophilised lactoferrin (Sochim Int. S.r.L.) from bovine milk, CAS number 339615-76-8, total protein content no less than 95% weight of dried substance, total lactoferrin content: no less than 95% weight of dried substance, pH 5.3-7.5
- Sodium chloride (A.C.E.F. Italy): Ph. Eur, USP grade
- Sodium phosphate monobasic dihydrate (Sigma Aldrich), content 98-100.5% weight of dried substance, meets specification of Ph. Eur. BP, USP, FCC, and 339, pH 4.1-5.0 (20°C, 1 %); 4.2- 4.5 (20°C, 5%)
- Sodium phosphate dibasic dihydrate (Sigma Aldrich), content 98.5-100.5% weight of dried substance, meets specification of Ph. Eur., pH 9.0-9.2 (20°C, 5%)
- Sodium hydrate (A.C.E.F. Italy) Ph. Eur., content 97.0-100.5 %.

### Method of preparation of the composition according to the invention

### Step 1 -

Raw materials: demineralised water, hydroxypropyl methylcellulose, lactoferrin from bovine milk.

Procedure: measure out the water and pour it into the tank, switch on the stirrer, add the hypromellose and wait until complete solubilisation. Add the lactoferrin and wait until complete solubilisation.

### Step 2 -

Raw materials: EDTA disodium salt, MicroGlicin 50, sodium hydrate

Procedure: Weigh and add the ingredients in the sequence described, waiting for complete solubilisation each time.

### Step 3 -

Raw materials: sodium phosphate monobasic, sodium phosphate dibasic, sodium chloride
Procedure: weigh and add the buffer salts and the sodium chloride and wait for their complete solubilisation.
Filtration on 0.2-micron PES filter
The solution obtained must undergo filtration with a 0.2 µm filter using a peristaltic pump.

### FORMULATION 1 :

### (Total weight: 200 g)

| | | |
|---|---|---|
| Purified water | 98.411 % | 196.822 g |
| Hypromellose | 0.15 % | 0.30 g |
| Lactoferrin | 0.10 % | 0.20 g |
| MicroGlicin 50 | 0.004 % | 0.008 g |
| EDTA | 0.10 % | 0.20 g |
| Sodium hydroxide (q.s. to pH 7.2) | | |
| Sodium phosphate monobasic | 0.15 % | 0.80 g |
| Sodium Chloride | 0.63 % | 1.26 g |

### FORMULATION 2:

### (Total weight: 500 g)

| | | |
|---|---|---|
| Lactoferrin | 0.10% | 0.50 g |
| Hypromellose | 0.15% | 0.75 g |
| MicroGlicin 50 | 0.004 % | 0.02 g |
| EDTA | 0.10% | 0.50 g |
| Sodium hydroxide (q.b. pH 7.2) | 0.01 % | 0.05g |
| Sodium phosphate monobasic | 0.15% | 0.75 g |
| Sodium phosphate dibasic | 0.40% | 2.00 g |
| Sodium Chloride | 0.66 % | 3.30 g |
| Purified water | 98.426 % | 492.13 g |

### FORMULATION 3:

### (Total weight: 700 g)

| | | |
|---|---|---|
| Lactoferrin | 0.10% | 0.70 g |
| Hypromellose | 0.15% | 1.05 g |
| MicroGlicin 50 | 0.004 % | 0.028 g |
| EDTA | 0.30% | 2.10g |
| Sodium hydroxide (q.s. to pH 7.2) | 0.032 % | 0.225 g |
| Sodium phosphate monobasic | 0.15% | 1.05 g |
| Sodium phosphate dibasic | 0.40 % | 2.80 g |
| Sodium Chloride | 0.60 % | 4.20 g |
| Purified water | 98.264 % | 687.848 g |

### FORMULATION 4:

### (Total weight: 300 g)

| | | |
|---|---|---|
| Water | 98.786 % | 296.358 g |
| Lactoferrin | 0.10 % | 0.30g |
| Hypromellose | 0.10 % | 0.30 g |
| MicroGlicin 50 | 0.004 % | 0.012 g |
| EDTA | 0.10 % | 0.30 g |
| KOH (20% sol.) | 0.09 % | 0.27 g |
| Sodium phosphate monobasic | 0.15 % | 0.45 g |
| Sodium phosphate dibasic | 0.40 % | 1.20 g |
| NaCl | 0.27 % | 0.81 g |

### FORMULATION 5:

### (Total weight 500 g)

| | | |
|---|---|---|
| Lactoferrin | 0.10 % | 0.50 g |
| Hypromellose | 0.10 % | 0.50 g |
| MicroGlicin 50 | 0.004 % | 0.02 g |
| EDTA | 0.10% | 0.50 g |
| KOH | 0.012 % | 0.06 g |
| sodium phosphate monobasic | 0.15 % | 0.75 g |
| sodium phosphate dibasic | 0.40 % | 2.00 g |
| NaCl | 0.65 % | 3.25 g |
| water | 98.484 % | 494.29 g |

### FORMULATION 6 (further containing dipotassium glvcyrrhizate):

### (Total weight: 100 g)

| | |
|---|---|
| Lactoferrin | 0.10 g |
| Dipotassium glycyrrhizate | 0.5 g |
| Hypromellose | 0.10 g |
| MicroGlicin^{™} 50 | 0.004 g |
| EDTA disodium salt | 0.10 g |
| Sodium phosphate monobasic | 0.15 g |
| Sodium phosphate dibasic | 0.40 g |
| Sodium Chloride | 0.20 g |
| Purified water | 98.426 g |
| pH 7.10 ±0.20, viscosity 2.20 + 0.70 cSt | |

Dose: apply one or two drops in each eye one or more times a day or according to a physician's advice, by way of non-limiting example in order to obtain an action coadjuvating antiviral treatment, also in the presence of inflammatory states.

### Results

In formulations with polymers other than hypromellose and xanthan (0.15% weight/weight of the composition), it was observed that the polymer (ethylene oxide/propylene oxide block polymer bound with units of urethane, urea and allophanate, hyaluronic acid, carboxymethylcellulose and hydroxyethylcellulose) was degraded by lactoferrin, leading to a degradation of both substances and the formation of by-products of degradation.

The formulation according to the present invention, by contrast, is stable for at least 2 or more weeks.

Application in the veterinary field.

### Materials and methods

Over a 12-month period, 30 dogs and 30 cats admitted to a veterinary ophthalmology centre in Wiesbaden with an infectious corneal ulcer were included in an observational study on the application. In addition to local antibiotic therapy (moxifloxacin [Vigamox^{®} AT] or ofloxacin [Floxal^{®} EDO AT], × 4-6/die) and systemic antibiotic therapy (doxycycline, 5-10 mg/kg x2/die) and debridement of the cornea with a dry swab and subsequent chemical cauterisation of the ulcer with iodine solution (DAB8), in 15 dogs and 15 cats, eye drops containing lactoferrin and EDTA were additionally applied locally x3/die to inhibit the matrix metalloproteinase. 15 dogs and 15 cats received a2- macroglobulins and growth factors obtained from serum. All animals were examined and treated in the surgery at the beginning of each day or every other day; after the vascularisation of the corneal ulcer, the intervals were individually prolonged until complete healing. Animals with descemetocele (deep ulcer reaching the Descemet membrane) were included in the study after the owners had been thoroughly informed and only if vascularisation had already reached the edge of the ulcer or the owner in any case rejected surgical intervention.

### Results

In all patients complete healing of the infected ulcer was obtained within at most 3 months. In all cases, it was possible to block fusion of the cornea (keratomalacia), and in no case was surgical intervention necessary. In all cases the process of cornea vascularisation, which subsequently led to healing, began within at most one week.

### Discussion

According to the depth and cause that has provoked corneal ulceration, different types of healing processes may occur. The corneal epithelium is subject to continual renewal through basal cell mitosis, as well as cell migration from the limbus toward the centre of the cornea at a rate of 20-50 µ 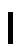 η/hour. This type of healing begins as early as 1 hour after a lesion, and the entire epithelium can be replaced in the space of 48-72 hours.

In the corneal stroma, on the other hand, it is possible to distinguish between avascular and vascular healing. As regards non-vascular healing, one observes the immigration of leukocytes from the tear fluid and aqueous humour as well as through migration from the limbus. The corneal cells - keratocytes - in the vicinity of the lesion die and one observes the transformation of the adjacent keratocytes into activated keratocytes. These fibroblasts produce collagen fibrils and the base substance, which, however, are arranged in an irregular manner, so that an opacification of the cornea occurs. After 48 hours, macrophages remove the dead material and the scar gradually becomes less dense.

In the case of vascular healing of the stroma, by contrast, one observes a much more intense cellular infiltration. Blood vessels form starting from the limbus and the granulation tissue (pannus) leads to a much denser scar. In principle, the stroma takes a lot longer to heal (years!) than the epithelium.

The normal physiological renewal of the cornea is influenced by many factors, including proteinase (first of all the matrix metalloproteinase, formerly defined as collagenase) and the inhibitors thereof (for example, the inhibitors of o1-protease and a2-macroglobulins from tear fluid, growth factors and cytokines). If there is a shift in the balance towards proteinase, a pathological degeneration of the stroma will ensue. Microorganisms as well as leukocytes and macrophages are capable of producing and releasing these proteolytic enzymes. In this case, one speaks of keratomalacia with a consequent gelatinous appearance of the cornea, often yellowish and protuberant. In order to counter this process of fusion of the cornea it is extremely important to contain the formation of proteinase as well as to combat the cause of infection. Pending arrival of the results of the bacteriological analysis, including the antibiogram, treatment should take place at a local level with wide-spectrum antibiotics (DNA gyrase inhibitors such as ofloxacin, ciprofloxacin, moxifloxacin or aminoglycosides such as gentamicin or tobramycin). In order to inhibit matrix metalloproteinases and serine proteases, autologous serum, N-acetylcysteine, Na-EDTA or tetracyclines can be used locally. Tetracyclines taken orally can also develop an antiproteolytic activity at the level of the tear fluid and cornea (doxycycline 10 mg/kg x1/die orally). In any case, it is advisable to combine together different protease inhibitors.

### Conclusions

Through the composition with EDTA and lactoferrin, the eye drops offer an easy-to-apply option for proteinase inhibition. Unlike autologous serum, eye drops can be easily kept in store and easily applied also in very small or uncooperative subjects. No hypersensitivities were observed in any patient. Thanks to the long-lasting effect, application every hour is not necessary as is generally required for other products. In all subjects, the eye drops were administered only 4 times a day.

Antimicrobial activity of the components against Staphylococcus aureus and Pseudomonas aeruginosa.

Determination of the minimum inhibiting concentration.

Products considered:
Sample No.1 : Lactoferrin 0.1 % + phosphate buffer
Sample No. 2: Lactoferrin 0.1% + phosphate buffer+EDTA 0.1 %
Strains tested:
   Staphylococcus aureus ATCC 6538
   Pseudomonas aeruginosa ATCC 9027

### Culture media:

- Tryptic Soy Broth (Liofilchem s.r.l)
- Tryptic Soy Agar (Liofichem s.r.l)

### Strain Preparation:

The above-described strains were adapted in the respective culture media specified above, incubation at 37°C for 3 days

### Test of Strain Viability:

The aim of the procedure was to obtain information about microbiological content through 1 :10 strain dilutions, by counting the viable colonies in Tryptic Soy Agar (incubation at 37°C for 3 days).

### Preparation of suspensions of known concentration:

At the end of the above-described treatments, stock solutions of a known concentration were prepared from the viable strains of *Staphylococcus aureus* and *Pseudomonas aeruginosa,* cultured in the above-described media according to the method of the Official Italian Pharmacopeia 12th ed.

### Dilution method:

Serial dilutions (two-fold) of the antimicrobial agent to be tested were added to a series of test tubes containing the culture medium (Tryptic Soy Broth).

Each test tube is inoculated with a standard amount of the microorganism, equal to 0.1 ml at concentrations comprised between 10⁵ -10⁸. After 20 hours of incubation at 37°C, the test tubes are checked for the presence of visible bacterial growth (turbidity): the absence of visible turbidity in the culture medium indicates a complete inhibition of bacterial growth.

### Control of strain development:

Seeding in Tryptic Soy Broth without the antimicrobial agent, incubated at 37°C for 20 h
Results: Assessment of microbial growth by observing visible turbidity in the inoculated test tubes
Sample No, 1 : Lactoferrin 0.1 % + phosphate buffer

### Inoculum concentration:

| | CFU/ml |
|---|---|
| *Staphylococcus aureus* ATCC 6538 | 5.2x10⁷ |

Observation of test tubes after 20h of incubation at 37°C

### (Staphylococcus aureus ATCC 6538)

| Sample | Turbidity |
|---|---|
| Lactoferrin 0.1 % + phosphate buffer | Present |

### Legend

Presence of Turbidity= visible bacterial growth
Absence of Turbidity= Inhibition of visible bacterial growth

### CONCLUSIONS:

The tested product does not exhibit antibacterial activity

### Inoculum concentration:

| | CFU/ml |
|---|---|
| *Pseudomonas aeruginosa* ATCC 9027 | 1x10⁶ |

Observation of test tubes after 20h of incubation at 37°C

### (Pseudomonas aeruginosa ATCC 9027)

| Sample | Turbidity |
|---|---|
| Lactoferrin 0.1 % + phosphate buffer | Present |

### Legend

Presence of Turbidity= visible bacterial growth
Absence of Turbidity= Inhibition of visible bacterial growth

### CONCLUSIONS:

The tested product does not exhibit antibacterial activity
Sample N°2: Lactoferrin 0.1 % + Phosphate buffer+EDTA 0.1 %
Inoculum concentration:

### CFU/ml

| | |
|---|---|
| *Staphylococcus aureus* ATCC 6538 | 9x10⁸ |
| Observation of test tubes after 20h of incubation at 37°C | |
| (*Staphylococcus aureus* ATCC 6538) | |
| % Lactoferrin Turbidity | |
| Lactoferrin 0.1 %+ EDTA 0.1% | Absent |

### Legend

Presence of Turbidity= visible bacterial growth
Absence of Turbidity= Inhibition of visible bacterial growth

### CONCLUSIONS:

| MIC (Minimum Inhibiting Concentration) | Result % (g/100ml) |
|---|---|
| Lactoferrin 0.1 %+EDTA 0.1 % | 0.1 |

Comment: The product tested exhibits a good inhibiting activity against Staphylococcus

### RESULTS

### Inoculum concentration:

| | CFU/ml |
|---|---|
| *Pseudomonas aeruginosa* ATCC 9027 | 131x10⁶ |

Observation of test tubes after 20h of incubation at 37°C

### (Pseudomonas aeruginosa ATCC 9027)

| | |
|---|---|
| % Lactoferrin | Turbidity |
| Lactoferrin 0.1 %+EDTA0.1 % | Absent |

### Legend

Presence of Turbidity= visible bacterial growth
Absence of Turbidity= Inhibition of visible bacterial growth

### CONCLUSIONS:

| MIC (Minimum Inhibiting Concentration) | Result % (g/100ml) |
|---|---|
| Lactoferrin 0.1 %+EDTA 0.1 % | 0.1 |

### Comment: The product tested exhibits a good inhibiting activity against Pseudomonas aee.

In conclusion, the compositions comprising lactoferrin and EDTA have a greater inhibiting activity than compositions comprising only lactoferrin at the same concentration, demonstrating a synergistic interaction between EDTA and lactoferrin.

## Claims

1. A composition (C1) for use in the curative or preventive treatment of at least one pathology or disorder of the eye or of the periocular area, and of the symptoms thereof, comprising:
i. a mixture which comprises or, alternatively, consists of:
(a) hydroxypropyl methylcellulose;
(b) lactoferrin;
(c) ethylenediaminetetraacetic acid (EDTA) or a salt thereof, and, optionally,
ii. one or more excipients for pharmaceutical use;
wherein said at least one pathology or disorder is at least one among conjunctivitis of bacterial origin, blepharitis, a bacterial infection and dry eye syndrome caused by bacteria belonging to the species *Staphylococcus aureus* or *Pseudomona aeruginosa,* and wherein said use comprises administration of said composition to a subject on the ocular surface or in the periocular area.

2. The composition (C1) for use according to claim 1, which comprises or, alternatively, consists of:
- (a) in an amount of 0.05% to 1%, preferably 0.10% to 0.15%;
- (b) in an amount of 0.05% to 1%, preferably 0.10% to 0.15%;
- (c) in an amount of 0.05% to 1%, preferably 0.10% to 0.15%; and/or
- ii., if present, in an amount of 0.05% to 1%, preferably 0.10% to 0.15%, wherein all of the percentages are by weight of the component relative to the total weight of (C1).

3. The composition (C1) for use according to any one of the preceding claims, which further comprises glycyrrhizic acid or salts thereof.

4. The composition (C1) for use according to any one of the preceding claims, wherein said use for preventive treatment comprises administration of said composition (C1) without additional substances with antibiotic action or said use for curative treatment comprises administration of said composition (C1) in combination with one or more substances with antibiotic action.

5. A pharmaceutical composition suitable for ophthalmic use or a medical device suitable for ophthalmic use comprising a composition containing:
i. a mixture which comprises, or, alternatively, consists of:
(a) hydroxypropyl methylcellulose;
(b) lactoferrin;
(c) ethylenediaminetetraacetic acid (EDTA) or a salt thereof, and, optionally,
ii. one or more excipients for pharmaceutical use;
wherein said at least one pathology or disorder is at least one among conjunctivitis of bacterial origin, blepharitis, a bacterial infection and dry eye syndrome caused by bacteria belonging to the species *Staphylococcus aureus* or *Pseudomona aeruginosa,* with the exclusion of a composition in the form of a cream for ophthalmic use comprising lactoferrin, EDTA and xanthan gum as component (a).

6. The pharmaceutical form or medical device according to claim 5, in the form of a liquid preparation such as, for example, an aqueous or oily collyrium, eye drops or suspension, a semi-solid preparation such as, for example, an unguent, ointment, gel or cream, or a solid preparation such as, for example, a powder, capsule, compress, ocular insert and the like.

7. A use of a composition containing:
i. a mixture comprising or, consisting of:
(a) hydroxypropyl methylcellulose;
(b) lactoferrin;
(c) ethylenediaminetetraacetic acid (EDTA) or a salt thereof, and, optionally,
ii. one or more excipients for pharmaceutical use,
to prevent or reduce the formation of biofilm caused by bacteria belonging to the species *Staphylococcus aureus* or *Pseudomona aeruginosa* on contact lenses.

## Patentansprüche

1. Zusammensetzung (C1) zur Verwendung bei der kurativen oder präventiven Behandlung mindestens einer Pathologie oder Störung des Auges oder des periokulären Bereichs und der Symptome davon, umfassend:
i. ein Gemisch, das Folgendes umfasst oder alternativ dazu aus Folgendem besteht:
(a) Hydroxypropylmethylcellulose;
(b) Lactoferrin;
(c) Ethylendiamintetraessigsäure (EDTA) oder ein Salz davon und gegebenenfalls
ii. einen oder mehrere Hilfsstoffe für pharmazeutische Zwecke,
wobei es sich bei der mindestens einen Pathologie oder Störung um mindestens eine von Konjunktivitis bakteriellen Ursprungs, Blepharitis, einer bakteriellen Infektion und dem Syndrom des trockenen Auges handelt, das durch Bakterien verursacht wird, die zu den Spezies der *Staphylococcus aureus* oder *Pseudomona aeruginosa* gehören, und wobei die Verwendung die Verabreichung der Zusammensetzung an ein Subjekt auf der Augenoberfläche oder im periokulären Bereich umfasst.

2. Zusammensetzung (C1) zur Verwendung nach Anspruch 1, umfassend oder alternativ bestehend aus:
- (a) in einer Menge von 0,05 % bis 1 %, vorzugsweise 0,10 % bis 0,15 %;
- (b) in einer Menge von 0,05 % bis 1 %, vorzugsweise von 0,10 % bis 0,15 %;
- (c) in einer Menge von 0,05 % bis 1 %, vorzugsweise von 0,10 % bis 0,15 %; und/oder
- ii., falls vorhanden, in einer Menge von 0,05 % bis 1 %, vorzugsweise von 0,10 % bis 0,15 %, wobei sich alle Prozentsätze auf das Gewicht der Komponente relativ zum Gesamtgewicht von (C1) beziehen.

3. Zusammensetzung (C1) zur Verwendung nach einem der vorhergehenden Ansprüche, die ferner Glycyrrhizinsäure oder Salze davon umfasst.

4. Zusammensetzung (C1) zur Verwendung nach einem der vorhergehenden Ansprüche, wobei die Verwendung zur präventiven Behandlung die Verabreichung der Zusammensetzung (C1) ohne zusätzliche Substanzen mit antibiotischer Wirkung umfasst oder die Verwendung zur kurativen Behandlung die Verabreichung der Zusammensetzung (C1) in Kombination mit einer oder mehreren Substanzen mit antibiotischer Wirkung umfasst.

5. Pharmazeutische Zusammensetzung, die zur ophthalmischen Verwendung geeignet ist, oder eine medizinische Vorrichtung, die zur ophthalmischen Verwendung geeignet ist, umfassend eine Zusammensetzung, die Folgendes enthält:
i. ein Gemisch, das Folgendes umfasst oder alternativ dazu aus Folgendem besteht:
(a) Hydroxypropylmethylcellulose;
(b) Lactoferrin;
(c) Ethylendiamintetraessigsäure (EDTA) oder ein Salz davon und gegebenenfalls
ii. einen oder mehrere Hilfsstoffe für pharmazeutische Zwecke,
wobei es sich bei der mindestens einen Pathologie oder Störung um mindestens eine von Konjunktivitis bakteriellen Ursprungs, Blepharitis, einer bakteriellen Infektion und dem Syndrom des trockenen Auges handelt, das durch Bakterien verursacht wird, die zu den Spezies der *Staphylococcus aureus* oder *Pseudomona aeruginosa* gehören, mit Ausnahme einer Zusammensetzung in Form einer Creme zur ophthalmischen Verwendung, die Lactoferrin, EDTA und Xanthangummi als Komponente (a) umfasst.

6. Pharmazeutische Form oder medizinische Vorrichtung nach Anspruch 5, in Form eines flüssigen Präparats, wie beispielsweise ein wässriges oder öliges Collyrium, Augentropfen oder -suspension, eines halbfesten Präparats, wie beispielsweise eine Salbe, ein Balsam, eine Gel oder eine Creme, oder eines festen Präparats, wie beispielsweise ein Pulver, eine Kapsel, eine Kompresse, ein Augeneinsatz und dergleichen.

7. Verwendung einer Zusammensetzung, enthaltend:
i. eine Mischung, die Folgendes umfasst oder daraus besteht:
(a) Hydroxypropylmethylcellulose;
(b) Lactoferrin;
(c) Ethylendiamintetraessigsäure (EDTA) oder ein Salz davon und gegebenenfalls
ii. einen oder mehrere Hilfsstoffe für pharmazeutische Zwecke,
zur Verhinderung oder Reduzierung der Bildung eines Biofilms, der durch Bakterien, die zu den Spezies der *Staphylococcus aureus* oder *Pseudomona aeruginosa* gehören, auf Kontaktlinsen verursacht wird.

## Revendications

1. Composition (C1) pour l'utilisation dans le traitement curatif ou préventif d'au moins une pathologie ou d'un trouble de l'œil ou de la région périoculaire, et de ses symptômes, comprenant :
i. un mélange qui comprend ou, alternativement, consiste en :
(a) hydroxypropylméthylcellulose ;
(b) lactoferrine ;
(c) acide éthylènediaminetétraacétique (EDTA) ou l'un de ses sels, et, facultativement,
ii. un ou plusieurs excipients à usage pharmaceutique ; dans laquelle ladite au moins une pathologie ou trouble est au moins une parmi la conjonctivite d'origine bactérienne, la blépharite, une infection bactérienne et le syndrome de l'œil sec causés par des bactéries appartenant à l'espèce *Staphylococcus aureus* ou *Pseudomona aeruginosa,* et dans laquelle ladite utilisation comprend l'administration de ladite composition à un sujet sur la surface oculaire ou dans la région périoculaire.

2. Composition (C1) pour l'utilisation selon la revendication 1, qui comprend ou, alternativement, consiste :
- (a) en une quantité de 0,05 % à 1 %, de préférence de 0,10 % à 0,15 % ;
- (b) en une quantité de 0,05 % à 1 %, de préférence de 0,10 % à 0,15 % ;
- (c) en une quantité de 0,05 % à 1 %, de préférence de 0,10 % à 0,15 % ; et/ou
- ii., si présent, en une quantité de 0,05 % à 1 %, de préférence de 0,10 % à 0,15 %, dans laquelle tous les pourcentages sont en poids du composant par rapport au poids total de (C1).

3. Composition (C1) pour l'utilisation selon l'une quelconque des revendications précédentes, qui comprend en outre de l'acide glycyrrhizique ou des sels de celui-ci .

4. Composition (C1) pour l'utilisation selon l'une quelconque des revendications précédentes, dans laquelle ladite utilisation pour un traitement préventif comprend l'administration de ladite composition (C1) sans substances supplémentaires à action antibiotique ou ladite utilisation pour un traitement curatif comprend l'administration de ladite composition (C1) en combinaison avec une ou plusieurs substances à action antibiotique.

5. Composition pharmaceutique adaptée à un usage ophtalmique ou dispositif médical adapté à un usage ophtalmique, comprenant une composition contenant :
i. un mélange qui comprend ou, alternativement, consiste en :
(a) hydroxypropylméthylcellulose ;
(b) lactoferrine ;
(c) acide éthylènediaminetétraacétique (EDTA) ou l'un de ses sels, et, facultativement,
ii. un ou plusieurs excipients à usage pharmaceutique ; dans laquelle ladite au moins une pathologie ou trouble est au moins une parmi la conjonctivite d'origine bactérienne, la blépharite, une infection bactérienne et le syndrome de l'œil sec causés par des bactéries appartenant à l'espèce *Staphylococcus aureus* ou *Pseudomona aeruginosa,* à l'exclusion d'une composition sous la forme d'une crème à usage ophtalmique comprenant de la lactoferrine, de l'EDTA et de la gomme xanthane en tant que composant (a).

6. Forme pharmaceutique ou dispositif médical selon la revendication 5, sous forme d'une préparation liquide telle que, par exemple, un collyre aqueux ou huileux, des gouttes ou une suspension pour les yeux, une préparation semi-solide telle que, par exemple, un onguent, une pommade, un gel ou une crème, ou une préparation solide telle que, par exemple, une poudre, une capsule, une compresse, un insert oculaire et similaire.

7. Utilisation d'une composition, contenant :
i. un mélange, comprenant ou consistant en :
(a) hydroxypropylméthylcellulose ;
(b) lactoferrine ;
(c) acide éthylènediaminetétraacétique (EDTA) ou l'un de ses sels, et, facultativement,
ii. un ou plusieurs excipients à usage pharmaceutique, pour prévenir ou réduire la formation de biofilm causée par des bactéries appartenant à l'espèce *Staphylococcus aureus* ou *Pseudomona aeruginosa* sur les lentilles de contact.
